# EUROPEAN PATENT APPLICATION

(11) **EP 2 913 046 A1**
(43) Date of publication of application: **02.09.2015**
(21) Application number: 13848453.0
(22) Date of filing: 16.10.2013
(51) Int. Cl.: A61K 8/92, A61K 8/03, A61K 8/02, A61Q 19/00

(54) **OIL-IN-WATER FORM ORGANOGEL COSMETIC COMPOSITION HAVING VARIABLE TRANSPARENCY**

(30) Priority: 23.10.2012 KR 20120118016
(71) Applicant: Genic Co., Ltd., Seocho-gu, Seoul 137-889 (KR)
(72) Inventor: YOO, Hyun-Oh, Seoul 137-930 (KR); KIM, Jong-Chul, Seoul 120-788 (KR); KWAK, Jae-Hoon, Hwaseong-si Gyeonggi-do 445-736 (KR); CHOI, Eun-Kyoung, Hwaseong-si Gyeonggi-do 445-787 (KR); PARK, Dong-Kyun, Hwaseong-si Gyeonggi-do 445-893 (KR)
(74) Representative: Vidon Brevets & Stratégie
(86) International application number: PCT/KR2013/009263
(87) International publication number: WO 2014/065536

(57) **Abstract**

The present invention relates to an oil-in-water form organogel cosmetic composition having variable transparency. More particularly, the oil-in-water (O/W) form organogel cosmetic composition comprising an oil phase part and a water phase part according to the present invention, said cosmetic composition comprises: 0.05 to 50 wt% of an oil phase part and 50 to 99.95 wt% of a water phase part, based on the total weight; and is characterized in that the difference between the refractive index of the water phase part and the oil phase part is 0.03 to 0.16. The present invention not only has i) outstanding formulation stability depending on the temperature and time, but ii) also becomes transparent from an opaque white color by adjusting the content of a component contained in the water phase part and the oil phase part when applied to the skin so as to allow the optimal application time in order to be easily identified. Therefore, the present invention can be usefully applied to the development of cosmetics such as a mask pack requiring a certain application time.

## Description

### [Technical Field]

The present disclosure relates to an oil-in-water (O/W) type organogel cosmetic composition having variable transparency, and more particularly, to an O/W type organogel cosmetic composition including an oil phase portion and a water phase portion, usable for producing a cosmetic mask pack of which an optimal application time of the organogel cosmetic composition varying according to the condition, temperature and humidity of a user's skin can be checked with the naked eye on the basis of the difference in refractive indices between the oil phase portion and the water phase portion.

### [Background Art]

Cosmetic packs are a type of basic cosmetic product, including products such as skin toners, lotions, and creams, attached to skin for a long period of time to maximize the supply of moisture and nutrients to the skin, unlike other cosmetic products absorbable by the skin if applied to the skin for a short period of time. Types of commercially available cosmetic packs include a mask type cosmetic pack which is manufactured by impregnating a nonwoven fabric with an essence liquid or forming a solid gel to be attached to and detached from the skin of the face, and a cream type cosmetic which can be uniformly applied to skin and then removed therefrom after a certain period of time by rinsing with water or by stripping solidified cosmetic materials off the skin. Various types of cosmetic packs may be used according to consumer preference.

Generally, compositions for such cosmetic packs are applied to the skin for a certain period of time to maximize the absorption of moisture and components in the skin, and are then removed from the skin. Since optimal application times of such compositions may be varied according to the conditions, temperature, and humidity of a user's skin, studies have constantly been undertaken in the related art to develop cosmetic compositions that can be checked with the naked eye to determine application times thereof.

Such studies are examined as follows. Although methods of mixing phenol-based pH indicators having functional groups discolored in an alkaline environment are disclosed in Japanese Patent Application Laid-open Publication No. 1982-48905, it is difficult to apply the phenol-based indicators to actual cosmetic compositions because such phenol-based indicators have problems in causing skin troubles. In addition, although oil-in-water (O/W) emulsion type cosmetic compositions for cosmetic packs consisting of an oil phase portion and a water phase portion that have a small difference in refractive indices are disclosed in Japanese Patent Application Laid-open Publication No. 1995-82129, United States Patent No. 6007799, it is difficult to control the time periods during which the cosmetic compositions change from transparency to ivory white because the technique of using a difference in refractive indices is not applicable.

Therefore, in the related art, research is required into developing cosmetic compositions for cosmetic packs that can be checked with the naked eye to determine optimal application times thereof according to the conditions, temperature and humidity of a user's skin.

### [Related Art Documents]

Patent document 1: Japanese Patent Application Laid-open Publication No. 1982-48905
Patent document 2: Japanese Patent Application Laid-open Publication No. 1995-82129
Patent document 3: United States Patent No. 6007799

### [Disclosure]

### [Technical Problem]

The inventors have conducted research into developing a cosmetic composition for a pack that can be checked with the naked eye to determine an optimal application time according to the conditions, temperature and humidity of a user's skin. As results of the research, the inventors have found that an oil-in-water (O/W) type organogel cosmetic composition including an oil phase portion and a water phase portion that have a difference in refractive indices within the range of 0.03 to 0.16 has i) improved formulation stability, and ii) when the organogel cosmetic composition is applied to a user's skin, the color of the organogel cosmetic composition changes from opaque ivory white to be transparent as moisture of the organogel cosmetic composition evaporates so that a user may determine an optimal application time of the organogel cosmetic composition on the basis of the color change. On the basis of this knowledge, the inventors have invented the present invention.

Therefore, an aspect of the present disclosure may provide an O/W type organogel cosmetic composition whose optimal application time varying according to the temperature, humidity, and conditions of a user's skin can be easily determined with the naked eye.

### [Technical Solution]

According to an aspect of the present disclosure, an oil-in-water (O/W) type organogel cosmetic composition having variable transparency may include an oil phase portion and a water phase portion, wherein the oil phase portion may be contained in an amount of 0.05% by weight to 50.00% by weight on the basis of total weight, the water phase portion may be contained in an amount of 50% by weight to 99.95% by weight on the basis of total weight, and the oil phase portion and the water phase portion may have a difference in refractive indices within a range of 0.03 to 0.16.

According to an embodiment of the present disclosure, the organogel cosmetic composition may include 5% by weight to 20% by weight of the oil phase portion and 80% by weight to 95% by weight of the water phase portion on the basis of total weight.

According to an embodiment of the present disclosure, the oil phase portion and the water phase portion each may have a refractive index within a range of 1.350 to 1.550.

According to an embodiment of the present disclosure, the water phase portion may include a moisturizer, a gelling polymer, and purified water.

According to an embodiment of the present disclosure, the moisturizer may include a polyvalent alcohol, glycerin, a saccharide, or a mixture thereof, and may be contained in an amount of 1% by weight to 70% by weight on the basis of total compositional weight.

According to an embodiment of the present disclosure, the gelling polymer may include a natural saccharide, a hydrophilic gelling agent, or a mixture thereof, and may be contained in an amount of 0.1% by weight to 10% by weight on the basis of total compositional weight.

According to an embodiment of the present disclosure, the oil phase portion may include oil and a surfactant.

According to an embodiment of the present disclosure, the oil may include an ester-based oil, a hydrocarbon-based oil, a wax, a natural oil, a silicone oil, or a mixture thereof, and may be contained in an amount of 0.05% by weight to 50.00% by weight on the basis of total compositional weight.

According to an embodiment of the present disclosure, the surfactant may be contained in an amount of 10% or less by weight on the basis of total compositional weight, and the surfactant may include an ester-based nonionic surfactant of a polyvalent alcohol and a fatty acid, a nonionic surfactant of an aliphatic polyoxyethylene polymerization system, a polyoxyethylene ether-based nonionic surfactant of a sorbitan ester, a polyoxyethylene ether-based nonionic surfactant of a natural oil, a polyoxyethylene ether-based nonionic surfactant, a alkaline anionic surfactant, a neutral anionic surfactant, or a mixture thereof.

### [Advantageous Effects]

The present disclosure provides an oil-in-water (O/W) type organogel cosmetic composition having variable transparency, the organogel cosmetic composition including an oil phase portion and a water phase portion, wherein the oil phase portion is contained in an amount of 0.05% by weight to 50.00% by weight on the basis of total weight, the water phase portion is contained in an amount of 50% by weight to 99.95% by weight on the basis of total weight, and the oil phase portion and the water phase portion has a difference in refractive indices within a range of 0.03 to 0.16. The organogel cosmetic composition may be usefully utilized in the development of cosmetic products such as mask packs requiring predetermined application times because i) the organogel cosmetic composition not only has high formulation stability according to temperature and time, ii) but also enables an optimal application time to be easily determined with the naked eye by controlling the contents of components contained in the water phase portion and the oil phase portion in such a manner that the color of the organogel cosmetic composition may be changed from an opaque ivory white color to be transparent when the organogel cosmetic composition is applied to skin.

### [Best Mode]

Exemplary embodiments of the present disclosure will now be described in detail.

Exemplary embodiments of the present disclosure provide an oil-in-water (O/W) type organogel cosmetic composition having variable transparency, the organogel cosmetic composition including an oil phase portion and a water phase portion, wherein the oil phase portion is contained in an amount of 0.05% by weight to 50.00% by weight on the basis of total weight, the water phase portion is contained in an amount of 50% by weight to 99.95% by weight on the basis of total weight, and the oil phase portion and the water phase portion has a difference in refractive indices within a range of 0.03 to 0.16. Therefore, when the organogel cosmetic composition is applied to the skin, moisture from the organogel cosmetic composition evaporates at the temperature of the skin, and thus the difference in refractive indices between the oil phase portion and the water phase portion is decreased to render the organogel cosmetic composition transparent. As a result, an optimal application time of the organogel cosmetic composition may be easily determined with the naked eye according to a user's skin condition.

The O/W type organogel cosmetic composition in which the oil phase portion is dissolved in the water phase portion provides good moisturizing and oiling sensations. The cosmetic composition of the present disclosure may include 0.05% by weight to 50.00% by weight, preferably 5% by weight to 20% by weight of the oil phase portion on the basis of the total weight of the cosmetic composition. At this time, realization of an opaque ivory white color may be difficult due to an inadequate content of the oil phase portion if the cosmetic composition includes less than 0.05% by weight of the oil phase portion, and O/W type formulation stability may be lowered if the cosmetic composition includes more than 50.00% by weight of the oil phase portion. Further, the cosmetic composition of the present disclosure may include 50% by weight to 99.95% by weight, preferably 80% by weight to 95% by weight of the water phase portion on the basis of the total compositional weight. At this time, formation of an O/W type emulsion may be difficult if the cosmetic composition includes less than 50% by weight of the water phase portion, and realization of an opaque ivory white color may be difficult if the cosmetic composition includes more than 99.95% by weight of the water phase portion.

Furthermore, the O/W type organogel cosmetic composition of the present disclosure is characterized by having an opaque ivory white color when the difference in refractive indices between the water phase portion and the oil phase portion is within the range of 0.03 to 0.16, and changing to be transparent as the difference in refractive indices between the water phase portion and the oil phase portion is decreased when the organogel cosmetic composition is applied to skin and loses its moisture due to evaporation at the temperature of the skin. According to an embodiment of the present disclosure, the oil phase portion or the water phase portion may have a refractive index within the range of 1.350 to 1.550, and the difference in refractive indices therebetween may be 0.03 to 0.16, preferably 0.08. If the difference in refractive indices is less than 0.03, it may be difficult to realize an opaque ivory white color, and if the difference in refractive indices is greater than 0.16, the difference in refractive indices is too great to expect an effective change to be transparent through the evaporation of moisture at the temperature of skin.

Since the cosmetic composition of the present disclosure has variable transparency according to the difference in refractive indices between the water phase portion and the oil phase portion, an optimal application time of the cosmetic composition may be easily determined with the naked eye according to personal differences.

The difference in refractive indices between the water phase portion and the oil phase portion of the cosmetic composition of the present disclosure may be appropriately controlled within the above-mentioned range according to essential components, an application object, and a target application time of the cosmetic composition. At this time, in order to control the difference in refractive indices between the oil phase portion and the water phase portion, the contents of a moisturizer included in the water phase portion or oil added in the oil phase portion may be controlled. More specifically, a method of increasing the contents of polyvalent alcohols, glycerins, or saccharides included in the water phase portion, decreasing the contents of oil and other components included in the oil phase portion, or using oil with a low refractive index may be adopted to lower the difference in refractive indices. On the contrary, a method of decreasing the contents of polyvalent alcohols, glycerins, or saccharides in the water phase portion, or increasing the content or the refractive index of oil in the oil phase portion may be adopted to increase the difference in refractive indices.

In the organogel cosmetic composition of the present disclosure, the water phase portion may include a typical moisturizer, a gelling polymer, and purified water.

At this time, any moisturizer may be used as the moisturizer if the moisturizer includes typical cosmetic moisturizer components for supplying moisture to skin. For example, the moisturizer may include one or more selected from the group consisting of: polyvalent alcohols including butylenes glycol, propylene glycol, pentylene glycol, hexylene glycol, polyethylene glycol, etc; glycerins including glycerin, diglycerin, polyglycerin, etc; and saccharides including sorbitol maltitol, sucrose, starch sugar, lactitol, etc. However, the moisturizer is not limited thereto. The moisturizer may be contained in a range of 1% by weight to 70% by weight, preferably 5% by weight to 45% by weight of the total compositional weight. If the moisturizer is contained in a range of less than 1% by weight, the difference in refractive indices between the water phase portion and the oil phase portion may be excessively large such that it may be difficult to change the transparency of the cosmetic composition. If the moisturizer is contained in a range of more than 70% by weight, the difference in refractive indices between the water phase portion and the oil phase portion may become so small that it may be difficult to realize an ivory white color in the cosmetic composition.

Furthermore, any gelling polymer typically used for implementing an organogel type of a cosmetic composition may be used as the gelling polymer. For example, the gelling polymer may include one or more selected from the group consisting of: natural saccharides including galactomannan, glucomannan, guar gum, locust bean gum, Pluronic, agar, algin, carrageenan, xanthan, gellan, gelatin, methylcellulose, cellulose, carboxymethyl cellulose, and hydroxyethyl cellulose; and hydrophilic gelling agents including polyacrylate or its salts, polymethacrylic acid, carboxy vinyl polymer, polyvinyl alcohol, polyacrylamide, polyethyleneoxide, polyethyleneimine, and polyvinylpyrrolidone. However, the gelling polymer is not limited thereto. The gelling polymer may be included in a range of 0.1% by weight to 10% by weight, preferably 0.5% by weight to 5% by weight on the basis of the total weight of the cosmetic composition. If the gelling polymer is included in a range of less than 0.1% by weight, the flexibility of the cosmetic composition may be lowered due to gel hardening, and thus the cosmetic composition may exhibit a low degree of adhesion when applied to skin. If the gelling polymer is included in a range of more than 10% by weight, gel softening may excessively occur such that it may be difficult to maintain the type of the cosmetic composition, and elasticity of the cosmetic composition may be lowered.

Further, in the cosmetic composition of the present disclosure, the oil phase portion may include a typical oil and a surfactant.

At this time, any kind of oil typically used in a cosmetic composition may be used as the oil of the cosmetic composition of the present disclosure. For example, the oil may include one or more selected from the group consisting of: ester-based oils including isopropyl myristate, ethyl laurate, ethyl myristate, isopropyl palmitate, butyl laurate, hexyl laurate, caprylic/capric triglyceride, butyl glycol dicaprylate/dicaprate, isopropyl myristate, triethylhexanoin, cetyl ethylhexanoate, octyldodecanol, and polyol ester; hydrocarbon-based oils including cyclododecane, isooctane, liquid paraffin, Vaseline, hydrogenated polydecene, cyclooctane, cyclopentane, and squalane; waxes including microcrystalline wax, beeswax, lanoline wax, ozokerite wax, candelilla wax, carnauba wax, and synthetic wax; natural oils including jojoba oil, avocado oil, almond oil, olive oil, sesame oil, wheat germ oil, safflower oil, camellia oil, caster oil, grape seed oil, green tea seed oil, macadámia nut oil, palm oil, rose hips oil, hydrogenated oil, argan oil, sea buckthorn oil, argan tree kernel oil, and lavender oil; and silicone oils including cyclomethicone, phenyl trimethicone, cyclopentasiloxane, and dimethicone. However, the oil is not limited thereto. According to an embodiment of the present disclosure, the oil may include olive oil, dimethicone, caprylic/capric triglyceride, cetyl ethylhexanoate, liquid paraffin, or a mixture thereof. According to the present disclosure, the oil may be included in a range of 0.05% by weight to 50.00% by weight, preferably 5% by weight to 20% by weight on the basis of the total weight of the cosmetic composition. At this time, if the oil is included in a range of less than 0.05% by weight, realization of an ivory white color in the organogel cosmetic composition may be difficult. If the oil is included in a range of more than 50.00% by weight, formation of an O/W type emulsion in the organogel cosmetic composition may be difficult, and thus the formulation stability of the organogel cosmetic composition may be lowered.

Further, any kind of surfactant typically used to realize and maintain an O/W form of a cosmetic composition may be used as the surfactant. The surfactant may include one or more selected from the group consisting of: ester-based nonionic surfactants of polyvalent alcohols and fatty acids including sorbitan stearate, sorbitan laurate, sorbitan palmitate, glyceryl stearate, and polyglyceryl stearate; nonionic surfactants of fatty acid polyoxyethylene polymerization systems including polyoxyethylene stearylether and polyoxyethylene oleate; polyoxyethylene ether based nonionic surfactants of sorbitan esters including polyoxyethylene sorbitan stearate, polyoxyethylene sorbitan laurate, polyoxyethylene sorbitan oleate, and polyoxyethylene sorbitol hexaoleate; polyoxyethylene ether based nonionic surfactants of natural oils including polyoxyethylene hydrogenated castor oil; polyoxyethylene ether based nonionic surfactants of polyoxypropylene including Poloxamer 168 and Poloxamer 407; alkaline anionic surfactants including sodium stearate, potassium stearate, and sodium laurate; and neutral anionic surfactants including sodium lauryl sulfate, sodium laureth sulfate, ammonium laureth sulfate, and potassium cetyl phosphate. However, the surfactant is not limited thereto. The surfactant may be included in a range of 10% by weight or less, preferably 5% by weight or less on the basis of the total compositional weight. At this time, if the surfactant is included in a range of more than 10% by weight, the surfactant may cause skin irritations and may hinder the gelation of the gelling polymer.

The O/W type organogel cosmetic composition of the present disclosure may additionally include a typical pH control agent, a preservation agent, a fragrance, a pigment, a disinfectant, an oxidation stabilizer, or a pearl agent in an amount that does not lower the effects of the cosmetic composition.

The O/W type organogel cosmetic composition of the present disclosure may be prepared by a method of preparing typical O/W form organogel cosmetic compositions. Specifically, the O/W type organogel cosmetic composition may be prepared by mixing components at room temperature or a high temperature to obtain a water phase portion mixture and an oil phase portion mixture, measuring the refractive indexes of the water phase portion mixture and the oil phase portion mixture, mixing the water phase portion mixture and the oil phase portion mixture at a high temperature, and cooling the water phase portion mixture and the oil phase portion mixture.

In an example of the present disclosure, components of an oil phase portion were mixed, and the mixed components were heated to 80°C to dissolve the components and thus obtain an oil phase portion mixture. In addition, components of a water phase portion were mixed and dissolved at room temperature to obtain a water phase portion mixture. The refractive indexes of the oil phase portion mixture and the water phase portion mixture were respectively measured. Thereafter, the water phase portion mixture and the oil phase portion mixture were stirred and mixed using a homomixer at a temperature of 70°C to 90°C and 3,000 rpm to 3,500 rpm for 15 to 20 minutes, and were degassed at a low pressure and cooled to 35°C. In this manner, an organogel cosmetic composition could be prepared.

According to the one or more of the exemplary embodiments of the present disclosure, (i) the organogel cosmetic composition has high formulation stability according to temperature and time, and ii) the organogel cosmetic composition can be controlled to exhibit transparency at a proper application time by adjusting the contents of components of the water phase portion and the oil phase portion. Therefore, when the organogel cosmetic composition is applied to a user's skin, since the color of the organogel cosmetic composition changes from an opaque ivory white to be transparent, an optimal application time for the user may be easily determined with the naked eye, and thus essential components may be maximally absorbed in the user's skin.

### [Mode for Invention]

Hereinafter, exemplary embodiments of the present disclosure are described more in detail through the following examples and comparative examples. However, the examples are provided for illustrative purposes only, and it will be apparent to those of ordinary skill in the art that the scope of the present invention is limited to the examples.

### Examples 1 to 5

According to compositions of the following Table 1, respective components of oil phase portions were mixed, the mixtures were heated to 80°C to dissolve the mixtures, and respective components of water phase portions were mixed and dissolved at room temperature. Respective refractive indexes of such mixed water phase portions and oil phase portions were measured using Abbe refractometers (A. KRUSS Optronic, Japan). After performing the refractive index-measuring process, the water phase portions and oil phase portions corresponding to respective examples were stirred and mixed at temperature and rotation speed conditions of 70°C to 90°C and 3,000 rpm to 3,500 rpm for 15 to 20 minutes by using a homomixer. Stirred oil-in-water (O/W) emulsions obtained as described above were degassed at a low pressure and cooled to 35°C to obtain organogel cosmetic compositions of Examples 1 to 5.

**[Table 1]**

| Classification | Composition (wt %) | Example 1 | Example 2 | Example 3 | Example 4 | Example 5 |
|---|---|---|---|---|---|---|
| oil phase | Cetyl ethylhexanoate, | 8.80 | 8.80 | 9.60 | 0.4 | 44.40 |
| | Dimethicone | 0.80 | 0.80 | - | - | 4.00 |
| | Polysorbate 60 | 0.30 | 0.30 | 0.30 | - | 1.50 |
| | Propyleneparaben | 0.10 | 0.10 | 0.10 | 0.10 | 0.10 |
| | Total | 10 | 10 | 10 | 0.5 | 50 |
| Water phase | Carrageenan | 1.00 | 1.00 | 1.00 | 1.00 | 1.00 |
| | Locust Bean Gum | 1.50 | 1.50 | 1.50 | 1.50 | 1.50 |
| | Glycerin | 28.30 | 50.30 | 8.30 | 31.80 | 15.30 |
| | Butylene glycol | 6.00 | 8.00 | 2.00 | 7.00 | 3.00 |
| | Methylparaben | 0.20 | 0.20 | 0.20 | 0.20 | 0.20 |
| | Purified water | 53.00 | 29.00 | 77.00 | 58.00 | 29.00 |
| | Total | 90 | 90 | 90 | 99.5 | 50 |

The refractive indexes of the oil phase portions and water phase portions of the organogel cosmetic compositions of Examples 1 to 5, difference in refractive indices between the oil phase portions and water phase portions, and weight ratios of the oil phase portions and water phase portions are shown in Table 2 below.

**[Table 2]**

| | Example 1 | Example 2 | Example 3 | Example 4 | Example 5 |
|---|---|---|---|---|---|
| Water phase portion's refractive index | 1.395 | 1.445 | 1.350 | 1.395 | 1.395 |
| Oil phase portion's refractive index | 1.475 | 1.475 | 1.500 | 1.475 | 1.475 |
| Difference in refractive indices between the oil phase portions and water phase portions | 0.080 | 0.030 | 0.160 | 0.08 | 0.08 |

### Comparative Examples 1 to 4

Cosmetic compositions of Comparative Examples 1 to 4 were prepared according to compositions shown in Table 3 below by the same method as that used in Examples 1 to 5.

**[Table 3]**

| Classification | Composition (wt.%) | Comparative Example 1 | Comparative Example 3 | Comparative Example 3 | Comparative Example 4 |
|---|---|---|---|---|---|
| Oil phase | Cetyl ethylhexanoate | 8.80 | - | 0.2 | 53.10 |
| | Phenyl trimethicone | - | 9.60 | - | - |
| | Dimethicone | 0.80 | - | - | 5.00 |
| | Polysorbate 60 | 0.30 | 0.30 | - | 1.80 |
| | Propyleneparaben | 0.10 | 0.10 | 0.10 | 0.10 |
| | Total | 10 | 10 | 0.3 | 60 |
| Water phase | Carrageenan | 1.00 | 1.00 | 1.00 | 1.00 |
| | Locust Bean Gum | 1.50 | 1.50 | 1.50 | 1.50 |
| | Glycerin | 55.30 | 2.30 | 31.90 | 12.80 |
| | Butylene glycol | 8.00 | 1.00 | 7.00 | 2.5 |
| | Methylparaben | 0.20 | 0.20 | 0.20 | 0.20 |
| | Purifies water | 24.00 | 84.00 | 58.10 | 22.00 |
| | Total | 90 | 90 | 99.7 | 40 |

The refractive indexes of oil phase portions and water phase portions of the cosmetic compositions of Comparative Examples 1 to 4, difference in refractive indices between the oil phase portions and water phase portions, and weight ratios of the oil phase portions and water phase portions are shown in Table 4 below.

**[Table 4]**

| | Comparative Example 1 | Comparative Example 2 | Comparative Example 3 | Comparative Example 4 |
|---|---|---|---|---|
| Water phase portions's refractive index | 1.465 | 1.535 | 1.395 | 1.395 |
| Oil phase portion's refractive index | 1.475 | 1.335 | 1.475 | 1.475 |
| Difference in refractive indices between the oil phase portions and water phase portions | 0.010 | 0.2 | 0.08 | 0.08 |

### Test Examples

The degrees of stability and white-to-transparency change times of the cosmetic compositions prepared in Examples 1 to 5 and Comparative Examples 1 to 4 were evaluated as follows.

### 1) Stability evaluation

After storing the cosmetic compositions at conditions of room temperature, 35°C, 50°C, and cycling atmosphere (-4°C to 45°C/day) for one week, two weeks, one month, two months, or three months, the degrees of formulation stability of the cosmetic compositions stored at the conditions were respectively observed with the naked eye and evaluated on the basis of the following evaluation standard. Evaluation results are shown in Tables 5 and 6.

### [Evaluation standard]

⊚ : No separation △ : Partial separation
× : Severe separation

**[Table 5]**

| Examples | Temperature | 1 week | 2 weeks | 1 month | 2 months | 3 months |
|---|---|---|---|---|---|---|
| Example 1 | Room temperature | ⊚ | ⊚ | ⊚ | ⊚ | ⊚ |
| | 35°C | ⊚ | ⊚ | ⊚ | ⊚ | ⊚ |
| | 50°C | ⊚ | ⊚ | ⊚ | ⊚ | ⊚ |
| | Cycling | ⊚ | ⊚ | ⊚ | ⊚ | ⊚ |
| Example 2 | Room temperature | ⊚ | ⊚ | ⊚ | ⊚ | ⊚ |
| | 35°C | ⊚ | ⊚ | ⊚ | ⊚ | ⊚ |
| | 50°C | ⊚ | ⊚ | ⊚ | ⊚ | ⊚ |
| | Cycling | ⊚ | ⊚ | ⊚ | ⊚ | ⊚ |
| Example 3 | Room temperature | ⊚ | ⊚ | ⊚ | ⊚ | ⊚ |
| | 35°C | ⊚ | ⊚ | ⊚ | ⊚ | ⊚ |
| | 50°C | ⊚ | ⊚ | ⊚ | ⊚ | |
| | Cycling | | | | | |
| Example 4 | Room temperature | | | | | |
| | 35°C | | | | | |
| | 50°C | | | | | |
| | Cycling | | | | | |
| Example 5 | Room temperature | | | | | |
| | 35°C | | | | | |
| | 50°C | | | | | |
| | Cycling | | | | | |

**[Table 6]**

| | | | | | | |
|---|---|---|---|---|---|---|
| Comparative Example 1 | Room temperature | | | | | |
| | 35°C | | | | | |
| | 50°C | | | | | |
| | Cycling | | | | | |
| Comparative Example 2 | Room temperature | | | | | |
| | 35°C | | | | | |
| | 50°C | | | | | |
| | Cycling | | | | | |
| Comparative Example 3 | Room temperature | | | | | |
| | 35°C | | | | | |
| | 50°C | | | | | |
| | Cycling | | | | | |
| Comparative Example 4 | Room temperature | | × | × | × | × |
| | 35°C | | × | × | × | × |
| | 50°C | × | × | × | × | × |
| | Cycling | | × | × | × | × |

It was confirmed that separation of the oil phase portion and the water phase portion was not found, and stability was maintained in the case of all of the cosmetic compositions of Examples 1 to 5 and the cosmetic compositions of Comparative Examples 1 to 3 stored at the conditions of room temperature, 35°C, 50°C, and cycling atmosphere (-4°C to 45°C/day). On the other hand, in case of the cosmetic composition of Comparative Example 4, separation of the water phase portion and the oil phase portion was observed, and it was confirmed that formulation stability was markedly lowered even under cycling conditions having various temperature variations.

### Evaluation of transparency variations

Transparency variations of the cosmetic compositions were evaluated according to a transparency change evaluating standard (evaluation values: 1 - opaque, 2-slightly opaque, 3 - moderate, 4 - good, 5 - excellent) by observing the extents to which the cosmetic compositions were changed from an ivory white color to be transparent with the naked eye in 1, 5, 10, 15, 20, 30, 40, 50, or 60 minutes after applying the cosmetic compositions of Examples 1 to 5 and Comparative Examples 1 to 4 to the skins of fifty healthy adult men and women. The mean values of results of the evaluation are shown in Table 7.

**[Table 7]**

| | Mean values | | | | | | | | |
|---|---|---|---|---|---|---|---|---|---|
| | 1 min | 5 min | 10 min | 15 min | 20 min | 30 min | 40 min | 50 min | 1 hr |
| Example 1 | 1.00 | 1.3 | 1.9 | 2.98 | 3.43 | 4.44 | 5.00 | 5.00 | 5.00 |
| Example 2 | 2.98 | 3.45 | 4.75 | 5.00 | 5.00 | 5.00 | 5.00 | 5.00 | 5.00 |
| Example 3 | 1.00 | 1 | 1. 60 | 2.12 | 3.12 | 3.58 | 3.98 | 4.12 | 4.85 |
| Example 4 | 1.00 | 1.27 | 2.13 | 3.22 | 3.52 | 4.54 | 5.00 | 5.00 | 5.00 |
| Example 5 | 1.00 | 1.32 | 2.34 | 3.32 | 3.64 | 4.62 | 5.00 | 5.00 | 5.00 |
| Comparative Example 1 | 5.00 | 5.00 | 5.00 | 5.00 | 5.00 | 5.00 | 5.00 | 5.00 | 5.00 |
| Comparative Example 2 | 1.00 | 1.00 | 1.00 | 1.23 | 1.45 | 1. 99 | 2.21 | 2.48 | 2.85 |
| Comparative Example 3 | 4.80 | 5.00 | 5.00 | 5.00 | 5.00 | 5.00 | 5.00 | 5.00 | 5.00 |
| Comparative Example 4 | 1.00 | 1.12 | 2.08 | 3.14 | 3.39 | 4.48 | 5.00 | 5.00 | 5.00 |

As a result, it was confirmed as seen in Table 7 that, although the organogel cosmetic compositions of Examples 1 to 5 of the present disclosure had different change times according to mixing ratios, the organogel cosmetic compositions were changed from an ivory white opaque color to be transparent with time. On the contrary, it was confirmed in case of Comparative Examples 1 to 4 that colors of the cosmetic compositions of Comparative Examples 1 and 3 were transparent from the beginning, the color of the cosmetic composition of Comparative Example 2 was barely changed to be transparent although the cosmetic composition of Comparative Example 2 was opaque, and the stability of the cosmetic composition (Comparative Example 4) was low although the cosmetic composition of Comparative Example 4 was changed from an opaque color to be transparent.

Hereby, it could be seen that the organogel cosmetic composition of the present disclosure can be controlled to exhibit transparency at an appropriate application time by adjusting the contents of components contained in the water phase portion and the oil phase portion of the organogel cosmetic composition, and it can be seen that the formulation stability of the organogel cosmetic composition according to temperature and time is high.

While exemplary embodiments have been shown and described above, it will be apparent to those skilled in the art that modifications and variations could be made without departing from the scope of the present invention as defined by the appended claims. The exemplary embodiments should be considered in descriptive sense only and not for purposes of limitation. Therefore, the scope of the invention is defined not by the detailed description but by the appended claims, and all differences within the scope will be construed as being included in the present invention.

## Claims

1. An oil-in-water (O/W) type organogel cosmetic composition having variable transparency, the organogel cosmetic composition comprising an oil phase portion and a water phase portion, wherein the oil phase portion is contained in an amount of 0.05% by weight to 50.00% by weight on the basis of total weight, the water phase portion is contained in an amount of 50% by weight to 99.95% by weight on the basis of total weight, and the oil phase portion and the water phase portion has a difference in refractive indices within a range of 0.03 to 0.16.

2. The organogel cosmetic composition of claim 1, comprising 5% by weight to 20% by weight of the oil phase portion and 80% by weight to 95% by weight of the water phase portion on the basis of total weight.

3. The organogel cosmetic composition of claim 1, wherein the oil phase portion and the water phase portion each have a refractive index within a range of 1.350 to 1.550.

4. The organogel cosmetic composition of claim 1, wherein the water phase portion comprises a moisturizer, a gelling polymer, and purified water.

5. The organogel cosmetic composition of claim 4, wherein the moisturizer comprises a polyvalent alcohol, glycerin, a saccharide, or a mixture thereof, and is contained in an amount of 1% by weight to 70% by weight on the basis of total compositional weight.

6. The organogel cosmetic composition of claim 4, wherein the gelling polymer comprises a natural saccharide, a hydrophilic gelling agent, or a mixture thereof, and is contained in an amount of 0.1% by weight to 10% by weight on the basis of total compositional weight.

7. The organogel cosmetic composition of claim 1, wherein the oil phase portion comprises oil and a surfactant.

8. The organogel cosmetic composition of claim 7, wherein the oil comprises an ester-based oil, a hydrocarbon-based oil, a wax, a natural oil, a silicone oil, or a mixture thereof, and is contained in an amount of 0.05% by weight to 50.00% by weight on the basis of total compositional weight.

9. The organogel cosmetic composition of claim 7, wherein the surfactant is contained in an amount of 10% or less by weight on the basis of total compositional weight, and the surfactant is an ester-based nonionic surfactant of a polyvalent alcohol and a fatty acid, a nonionic surfactant of an aliphatic polyoxyethylene polymerization system, a polyoxyethylene ether-based nonionic surfactant of a sorbitan ester, a polyoxyethylene ether-based nonionic surfactant of a natural oil, a polyoxyethylene ether-based nonionic surfactant, a alkaline anionic surfactant, a neutral anionic surfactant, or a mixture thereof.
